# EUROPEAN PATENT APPLICATION

(11) **EP 4 275 485 A2**
(43) Date of publication of application: **15.11.2023**
(21) Application number: 23153254.0
(22) Date of filing: 25.01.2023
(51) Int. Cl.: A01H 4/00, A01G 22/00

(54) **METHOD FOR PRODUCING SAPLING OF WOODY PLANT, METHOD FOR PRODUCING NATURAL RUBBER, METHOD FOR PRODUCING PNEUMATIC TIRE, METHOD FOR PRODUCING RUBBER PRODUCT, AND HEVEA BRASILIENSIS SAPLING**

(30) Priority: 12.05.2022 JP 2022078921
(71) Applicant: Sumitomo Rubber Industries, Ltd., Kobe-shi, Hyogo 651-0072 (JP)
(72) Inventor: OKADA, Akari, Kobe-shi, Hyogo, 651-0072 (JP); TONO, Kaori, Kobe-shi, Hyogo, 651-0072 (JP)
(74) Representative: J A Kemp LLP

(57) **Abstract**

The present invention provides a method for producing a sapling of a woody plant which enables the production of a sapling of a woody plant from a woody plant shoot-derived plantlet with high productivity, as well as a method for producing a natural rubber, a method for producing a pneumatic tire, and a method for producing a rubber product each of which includes the method for producing a sapling of a woody plant. Included is a method for producing a sapling of a woody plant including a cultivation step of cultivating a woody plant shoot-derived plantlet in a solid medium containing at least one medium component.

## Description

### TECHNICAL FIELD

The present invention relates to a method for producing a sapling of a woody plant, a methods for producing a natural rubber, a method for producing a pneumatic tire, a methods for producing a rubber product, and a *Hevea brasiliensis* sapling.

### BACKGROUND ART

At present, natural rubber (a type of polyisoprenoid) used in industrial rubber products is obtained by cultivating rubber-producing plants, such as Para rubber trees (*Hevea brasiliensis*) of the family *Euphorbiaceae* or Indian rubber trees (*Ficus elastica*) among *Moraceae* plants, so that the laticifer cells of these plants biosynthesize natural rubber, and manually harvesting the natural rubber from the plants.

Natural rubber for industrial applications is currently sourced almost entirely from *Hevea brasiliensis.* Moreover, it is used widely and in large amounts in a variety of applications as the main raw material for rubber products. However, *Hevea brasiliensis* is a plant that can be grown only in limited regions such as Southeast Asia and South America. Furthermore, *Hevea brasiliensis* requires about seven years from planting to becoming mature enough for rubber extraction, and the collection season may be limited. The time period during which natural rubber can be collected from the mature tree is also limited to 20 to 30 years.

In the future, an increase in the demand for natural rubber mainly in developing countries is expected, and the exhaustion of natural rubber resources is a matter of concern. Thus, a stable source of supply for natural rubber is desired.

Under these circumstances, efforts have been made to increase the production of natural rubber by *Hevea brasiliensis.* For *Hevea brasiliensis,* sapling propagation is carried out by raising and growing seedlings provided by seeding to yield a rootstock and grafting buds obtained from a clonal sapling to the rootstock. However, the grafts may be affected by the rootstock and thus fail to be true clonal saplings.

Moreover, micropropagation is known as a clonal sapling propagation technique using tissue culture. In the micropropagation technique, saplings are propagated by aseptic tissue culture. Specifically, a tissue, such as buds or shoot tips, taken from an individual plant to be propagated may be cultured to induce shoots, which may finally be rooted to produce plantlets. This approach can produce true clonal saplings.

### SUMMARY OF INVENTION

### TECHNICAL PROBLEM

Extensive studies of the present inventors have revealed that woody plant shoot-derived plantlets may insufficiently develop roots and are liable to result in delayed plant growth. Thus, when woody plant shoot-derived plantlets are to be commercially used, their root development and growth delay cause a major problem and there is room for improvement in producing a sapling of a woody plant from a woody plant shoot-derived plantlet with high productivity.

The present invention aims to solve the new problem discovered by the present inventors and provide a method for producing a sapling of a woody plant which enables the production of a sapling of a woody plant from a woody plant shoot-derived plantlet with high productivity, as well as a method for producing a natural rubber, a method for producing a pneumatic tire, and a method for producing a rubber product each of which includes the method for producing a sapling of a woody plant.

The present invention also aims to provide a novel *Hevea brasiliensis* sapling.

### SOLUTION TO PROBLEM

The present inventors have made extensive investigations in order to solve the new problem they had discovered, and then found that when a woody plant shoot-derived plantlet is cultivated in a solid medium containing a medium component, the rooting and root development, which tend to be slower than the growth of the above-ground parts, may be promoted to provide a good balance between the growth of the above-ground parts and root growth, and thus it is possible to produce a sapling of a woody plant from a woody plant shoot-derived plantlet with high productivity. This finding has led to the completion of the present invention.

Specifically, the present invention relates to a method for producing a sapling of a woody plant, including a cultivation step of cultivating a woody plant shoot-derived plantlet in a solid medium containing at least one medium component.

### ADVANTAGEOUS EFFECTS OF INVENTION

The method for producing a sapling of a woody plant of the present invention includes a cultivation step of cultivating a woody plant shoot-derived plantlet in a solid medium containing a medium component, and thus enables the production of a sapling of a woody plant from a woody plant shoot-derived plantlet with high productivity.

The method for producing a natural rubber of the present invention includes a plant production step of producing a sapling by the method for producing a sapling and thus enables the production of a natural rubber with high productivity.

The method for producing a pneumatic tire of the present invention includes producing a natural rubber by the method for producing a natural rubber and thus enables the production of a pneumatic tire with high productivity.

The method for producing a rubber product of the present invention includes producing a natural rubber by the method for producing a natural rubber and thus enables the production of a rubber product with high productivity.

The *Hevea brasiliensis* sapling of the present invention shows a high percentage of survival in soil and high growth in the young sapling stage.

### BRIEF DESCRIPTION OF DRAWINGS

FIG. 1 is a photograph showing one example in the vicinity of the root base.
FIG. 2 is a photograph showing one example having a joint.
FIG. 3 is a photograph showing one example having no joint.
FIG. 4 is a photograph showing one example which is taprooted in the vicinity of the root base.
FIG. 5 is a photograph showing one example which is not taprooted in the vicinity of the root base.
FIG. 6 is a photograph showing one example of cultivation of a plantlet in a solid medium (vermiculite supplemented with a liquid medium).
FIG. 7 is a photograph showing the state of the sapling of Example.
FIG. 8 is a photograph showing the state of the sapling of Comparative Example 1.
FIG. 9 is a photograph showing the state of the sapling of Comparative Example 2.

### DESCRIPTION OF EMBODIMENTS

### <Method for producing sapling of woody plant>

The method for producing a sapling of a woody plant (method for promoting the root growth of a woody plant) of the present invention includes a cultivation step of cultivating a woody plant shoot-derived plantlet in a solid medium containing a medium component. Thus, rooting and root development, which tend to be slower than the growth of the above-ground parts, may be promoted to provide a good balance between the growth of the above-ground parts and root growth, and thus it is possible to produce a sapling of a woody plant from a woody plant shoot-derived plantlet with high productivity.

It should be noted that as long as the method for producing a sapling of a woody plant of the present invention includes the above-described cultivation step, it may include other steps.

The reason why the present invention provides the above-mentioned advantageous effect is not clear, but is believed to be as follows.

When a woody plant shoot-derived plantlet is cultivated in a solid medium, the tangible solid medium will contact the roots and physically stimulate the roots (in particular, root caps), e.g., by contact or friction with the solid medium. As a result, the rooting and root growth and development are promoted. Thus, the contact between a tangible solid medium and roots promotes root growth.

Moreover, although, particularly in the initial stage of cultivation, a woody plant shoot-derived plantlet may show insufficient root growth and may not absorb sufficient necessary nutrients from the roots, the inclusion of a medium component in the solid medium allows the plantlet to absorb necessary nutrients from the roots, even if its root growth is insufficient. This promotes the root growth and also allows the above-ground parts to grow. Thus, the inclusion of a medium component in the solid medium promotes root growth and also allows the above-ground parts to grow.

As described above, due to the synergistic effect of the cultivation in a solid medium and the inclusion of a medium component in the solid medium, rooting and root development, which tend to be slower than the growth of the above-ground parts, may be promoted to provide a good balance between the growth of the above-ground parts and root growth, and thus it is possible to produce a sapling of a woody plant from a woody plant shoot-derived plantlet with high productivity.

In the micropropagation technique, a tissue, such as buds, taken from an individual plant to be propagated may be cultured in vitro to induce shoots, which may finally be rooted to produce plantlets. Then, the plantlets may be further grown to prepare saplings. Here, if the plantlets with insufficiently grown roots are cultivated in soil, the plantlets may grow significantly slowly or may die. Thus, in the conventional technique, plantlets are cultured in vitro after rooting for one to several months until their roots have elongated to some extent, before they are cultivated in soil.

In contrast, in the above-described production method, the medium component in the solid medium can promote the root growth of, for example, even a very young woody plant shoot-derived plantlet soon after rooting in vitro, and thus it is possible to greatly (even by one to several months) accelerate the time to shift from in vitro culture to cultivation in soil or the like as compared to the conventional approach, and also to grow a woody plant shoot-derived plantlet in a shorter period of time, thereby producing a sapling of a woody plant from the woody plant shoot-derived plantlet with high productivity. The production method does not require in vitro culturing of a plantlet for one to several months after rooting as in the conventional technique and thus can reduce the production period even by at least one to several months.

Moreover, a sapling of a woody plant produced by the production method has a good balance between the growth of the above-ground parts and root growth, and further its main root and lateral roots also develop well. Thus, the sapling shows a high percentage of survival in soil and high growth in the young sapling stage.

Herein, the term "shoot" refers to an apical bud, an axillary bud, or an adventitious bud and also refers to a bud differentiated from a multiple shoot or shoot primordia or any of the above-mentioned buds which have elongated.

Herein, the term "plantlet" refers to a rooted shoot. Here, the term "plantlet" herein encompasses, in addition to a rooted shoot, a rooted shoot that has shown a tendency of poor growth in soil cultivation.

Herein, the term "above-ground part" refers to a stem or a leaf on a stem.

Although the production method of the present invention is applicable to any plant (plant from which a shoot is derived), the plant is preferably a woody plant.

Non-limiting examples of the woody plant include a wide range of varieties and species of deciduous trees and evergreen trees. In particular, rubber trees from which rubber can be collected as a resource are preferred. More preferred are the genus *Hevea,* e.g., *Hevea brasiliensis,* the genus *Ficus,* e.g., *Ficus carica, Ficus elastica, Ficus pumila* L., *Ficus erecta* Thumb., *Ficus ampelas* Burm. f., *Ficus benguetensis* Merr., *Ficus irisana* Elm., *Ficus microcarpa* L. f., *Ficus septica* Burm. f., and *Ficus benghalensis,* and *Parhenium argentatum.* Still more preferred are plants belonging to the family *Euphorbiaceae,* e.g., the genus *Hevea,* particularly preferably plants belonging to the genus *Hevea. Hevea brasiliensis* is most preferred among these.

Examples of materials that may be used to induce the shoot include plant tissues such as plant petioles, laminas, hypocotyls of somatic embryos, nodes, axillary buds, and apical buds. Tissues including nodes, axillary buds, or apical buds are preferred among these because shoots can be stably induced therefrom. Specific examples include the above-mentioned tissues derived from mature trees, young trees, saplings, or clonal saplings and those derived from aseptic seedlings grown in vitro from seedlings.

When the above-mentioned tissues derived from mature trees, young trees, saplings, or clonal saplings are used, they may be cut to the required size as appropriate, followed by disinfection or sterilization of the surface before use. When the above-mentioned tissues derived from aseptic seedlings grown in vitro from seedlings are used, they may be cut to the required size as appropriate before use.

When the above-mentioned tissues derived from mature trees, young trees, saplings, or clonal saplings are used, the surface of the tissue is first cleaned before culture in a below-mentioned induction medium. For example, the surface may be cleaned with an abrasive powder or a soft sponge, but is preferably cleaned with running water. The water used for cleaning may contain approximately 0.1% by mass of surfactant(s).

Next, the tissue is disinfected or sterilized. The disinfection or sterilization may be carried out using known disinfectants or sterilizing agents, preferably ethanol, benzalkonium chloride, or an aqueous sodium hypochlorite solution. Here, the disinfection or sterilization treatment may further be followed by washing with sterile water.

As a specific example, the cleaning and disinfection or sterilization treatment may be carried out by the following procedure: clean the tissue surface with running water and wash the tissue with ethanol; then sterilize the tissue using an aqueous sodium hypochlorite solution optionally with stirring; and thereafter wash the tissue with sterile water.

Although the method for inducing a shoot is not limited, the following describes an exemplary induction step of inducing a shoot from the tissue or other material.

### (Induction step)

An induction step includes culturing the tissue in an induction medium containing a plant hormone and a carbon source to induce and form a shoot. Here, although the induction medium may be a liquid or a solid, solid culture is preferred because shoot induction is facilitated when the tissue is inserted into and cultured in a medium. Moreover, when the induction medium is a liquid medium, static culture or shake culture may be carried out.

Moreover, when a disinfected or sterilized tissue is used, the cut end is preferably cut off to eliminate the effect of the disinfectant or sterilizing agent before culture.

Examples of the plant hormone (plant growth hormone) include auxin plant hormones and/or cytokinin plant hormones. Cytokinin plant hormones are preferred among these.

Examples of the auxin plant hormones include 2,4-dichlorophenoxyacetic acid, 1-naphthaleneacetic acid, indole-3-butyric acid, indole-3-acetic acid, indolepropionic acid, chlorophenoxyacetic acid, naphthoxyacetic acid, phenylacetic acid, 2,4,5-trichlorophenoxyacetic acid, para-chlorophenoxyacetic acid, 2-methyl-4-chlorophenoxyacetic acid, 4-fluorophenoxyacetic acid, 2-methoxy-3,6-dichlorobenzoic acid, 2-phenyl acid, picloram, and picolinic acid. Among these, 2,4-dichlorophenoxyacetic acid, 1-naphthaleneacetic acid, and indole-3-butyric acid are preferred, and 2,4-dichlorophenoxyacetic acid or 1-naphthaleneacetic acid is more preferred.

Examples of the cytokinin plant hormones include benzyladenine, kinetin, zeatin, benzylaminopurine, isopentenylaminopurine, thidiazuron, isopentenyladenine, zeatin riboside, and dihydrozeatin. Among these, benzyladenine, kinetin, and zeatin are preferred, benzyladenine and kinetin are more preferred, and benzyladenine is still more preferred.

Any carbon source may be used, including sugars such as sucrose, glucose, trehalose, fructose, lactose, galactose, xylose, allose, talose, gulose, altrose, mannose, idose, arabinose, apiose, mannitol, sorbitol, xylitol, erythritol, and maltose. Sucrose is preferred among these.

The induction medium preferably further contains activated carbon in order to prevent growth inhibitors from accumulating in the tissue. Moreover, the induction medium preferably further contains silver nitrate in order to promote shoot formation. Furthermore, the induction medium may contain coconut water (coconut milk) in order to promote shoot formation.

Examples of the induction medium include those prepared by adding plant hormones to base media, including: basal media such as White's medium (disclosed on pp. 20-36 of Shokubutsu Saibo Kogaku Nyumon (Introduction to Plant Cell Engineering), Japan Scientific Societies Press), Heller's medium (Heller R, Bot. Biol. Veg. Paris 14, 1-223 (1953)), SH medium (Schenk and Hildebrandt medium), MS medium (Murashige and Skoog medium) (disclosed on pp. 20-36 of Shokubutsu Saibo Kogaku Nyumon (Introduction to Plant Cell Engineering), Japan Scientific Societies Press), LS medium (Linsmaier and Skoog medium) (disclosed on pp. 20-36 of Shokubutsu Saibo Kogaku Nyumon (Introduction to Plant Cell Engineering), Japan Scientific Societies Press), Gamborg medium, B5 medium (disclosed on pp. 20-36 of Shokubutsu Saibo Kogaku Nyumon (Introduction to Plant Cell Engineering), Japan Scientific Societies Press), MB medium (disclosed on pp. 222-245 of Biotechnology in Agriculture and Forestry volume 5 (TreesII)), and WP medium (for woody plants), and modified basal media obtained by altering the composition of the foregoing basal media (the disclosure of each of the foregoing documents is herein incorporated by reference). Preferred among these are those prepared by adding plant hormones to MS medium, B5 medium, WP medium, or MB medium. More preferred are those prepared by adding plant hormones to MS medium, a modified MS medium obtained by altering the composition of the medium, MB medium, or a modified MB medium obtained by altering the composition of the medium.

When the induction medium is used as a solid medium, the medium may be solidified using a solidifying agent. Any solidifying agent may be used, including Japanese gelatin, gellan gum, agarose, Gelrite, agar, and Phytagel.

The suitable composition and culture conditions of the induction medium vary depending on the plant species and also vary depending on whether the medium is a liquid medium or a solid medium, but the composition is usually as follows, particularly for rubber trees.

The carbon source concentration in the induction medium is preferably at least 0.1% by mass, more preferably at least 1.0% by mass, still more preferably at least 3.0% by mass. The carbon source concentration is preferably not more than 10% by mass, more preferably not more than 9.0% by mass, still more preferably not more than 6.0% by mass. Herein, the term "carbon source concentration" refers to the concentration of sugars.

Preferably, substantially no auxin plant hormone is added to the induction medium. Specifically, the auxin plant hormone concentration in the induction medium is preferably not more than 1.0 mg/L, more preferably not more than 0.1 mg/L, still more preferably not more than 0.05 mg/L, particularly preferably not more than 0.01 mg/L.

When a cytokinin plant hormone is added to the induction medium, the cytokinin plant hormone concentration in the induction medium is preferably at least 0.01 mg/L, more preferably at least 0.1 mg/L, still more preferably at least 0.5 mg/L, particularly preferably at least 0.8 mg/L, most preferably at least 3.0 mg/L. The cytokinin plant hormone concentration is preferably not more than 8.0 mg/L, more preferably not more than 7.0 mg/L, still more preferably not more than 6.0 mg/L.

In particular, when the cytokinin plant hormone used is benzyladenine, the benzyladenine concentration is preferably 4.0 to 6.0 mg/L, most preferably 5.0 mg/L. Moreover, when the cytokinin plant hormone used is kinetin, the kinetin concentration is preferably 0.8 to 1.2 mg/L, most preferably 1.0 mg/L.

The activated carbon concentration in the induction medium is preferably at least 0.01% by mass, more preferably at least 0.03% by mass. The activated carbon concentration is preferably not more than 1.0% by mass, more preferably not more than 0.1% by mass.

The silver nitrate concentration in the induction medium is preferably at least 0.1 mg/L, more preferably at least 0.3 mg/L, still more preferably at least 0.5 mg/L. The silver nitrate concentration is preferably not more than 5.0 mg/L, more preferably not more than 3.0 mg/L.

The pH of the induction medium is preferably 4.0 to 10.0, more preferably 5.0 to 6.5, still more preferably 5.5 to 6.0.

Herein, the term "pH" for solid media refers to the pH of the medium that incorporates all the components except the solidifying agent.

The induction step is usually carried out in a controlled environment in which culture conditions such as temperature and light cycle are managed. The culture conditions may be selected as appropriate, but, for example, the culture temperature is preferably 0 to 40°C, more preferably 20 to 40°C, still more preferably 25 to 35°C. Culture may be carried out in the dark or light, but, for example, the light conditions may include a light cycle of 14 to 16 hours light at 12.5 µmol/m²/s. The culture duration is not limited, but culture for one to ten weeks is preferred, and culture for three to five weeks is more preferred.

When the induction medium is a solid medium, the solidifying agent concentration in the induction medium is preferably at least 0.1% by mass, more preferably at least 0.2% by mass. The solidifying agent concentration is preferably not more than 2.0% by mass, more preferably not more than 1.1% by mass, still more preferably not more than 0.8% by mass.

Among the conditions indicated above, it is particularly preferred that the plant hormone used is a cytokinin plant hormone, particularly benzyladenine or kinetin, at a concentration of 3.0 to 8.0 mg/L and the culture temperature is 25 to 35°C.

A shoot can be induced and formed by culturing the tissue in the induction medium as described above.

Although the method for obtaining a plantlet from the shoot is not limited, the following describes an exemplary method for obtaining a plantlet from the shoot.

The formed shoot may be directly subjected to the culture step described later. Preferably, the shoot is subjected to the following soaking step before the culture step. In this case, a plantlet tends to be more suitably obtained.

### (Soaking step)

A soaking step includes soaking the shoot in an auxin solution containing an auxin plant hormone.

Specifically, the shoot (e.g., a shoot segment of about 2 cm) obtained in the induction step or other step may be soaked in the auxin solution.

Preferably, the shoot is soaked in the auxin solution such that the end of the shoot segment, namely, the cut end of the shoot, is submerged in the auxin solution.

Moreover, the shoot may be soaked in the auxin solution while it is allowed to stand still or shaken.

The duration of the soaking step is preferably 24 hours or longer, more preferably 40 hours or longer, still more preferably 60 hours or longer, particularly preferably 70 hours or longer, but is preferably 168 hours or shorter, more preferably 150 hours or shorter, still more preferably 130 hours or shorter, particularly preferably 100 hours or shorter, most preferably 90 hours or shorter, further most preferably 80 hours or shorter. This can lead to a better rooting ratio.

The soaking step is preferably carried out in a controlled environment in which conditions such as temperature and light cycle are managed. For example, the temperature in the soaking step is preferably 0 to 40°C, more preferably 20 to 40°C, still more preferably 25 to 35°C. The soaking step may be carried out in the dark or light, but, for example, the light conditions may include a light cycle of 14 to 16 hours light at 5 to 20 µmol/m²/s.

The shoot to be subjected to the soaking step may be any shoot which may be formed by any method. For example, it may be the shoot obtained in the induction step or other step.

Moreover, the shoot to be subjected to the soaking step is preferably a shoot segment. For example, it is preferably a shoot segment cut out of the tissue (e.g., axillary bud) used as the material for inducing a shoot.

The shoot is also preferably a shoot which has been cultured in the induction medium for at most six months.

The length of the shoot (shoot segment) to be subjected to the soaking step is preferably 10 mm or longer, more preferably 15 mm or longer, still more preferably 20 mm or longer, but is preferably 100 mm or shorter, more preferably 80 mm or shorter, still more preferably 50 mm or shorter. This can lead to a better rooting ratio.

The auxin plant hormone contained in the auxin solution may be as described for the auxin plant hormones usable in the induction medium. Indole-3-butyric acid and 1-naphthaleneacetic acid are preferred among these, with a combination of indole-3-butyric acid and 1-naphthaleneacetic acid being preferred.

The auxin plant hormone concentration in the auxin solution is preferably not more than 15 mg/L, more preferably not more than 12 mg/L, but is preferably at least 1.0 mg/L, more preferably at least 3.0 mg/L, still more preferably at least 5.0 mg/L, particularly preferably at least 8.0 mg/L. This can lead to a better rooting ratio.

Here, when multiple auxin plant hormones are used, the auxin plant hormone concentration means the total concentration of the auxin plant hormones.

When the auxin plant hormone used is a combination of indole-3-butyric acid and 1-naphthaleneacetic acid,
the indole-3-butyric acid concentration in the auxin solution is preferably not more than 10 mg/L, more preferably not more than 7.5 mg/L, still more preferably not more than 6.0 mg/L, but is preferably at least 1.0 mg/L, more preferably at least 2.5 mg/L, still more preferably at least 3.0 mg/L, particularly preferably at least 4.0 mg/L, and
the 1-naphthaleneacetic acid concentration in the auxin solution is preferably not more than 10 mg/L, more preferably not more than 7.5 mg/L, still more preferably not more than 6.0 mg/L, but is preferably at least 1.0 mg/L, more preferably at least 2.5 mg/L, still more preferably at least 3.0 mg/L, particularly preferably at least 4.0 mg/L. This can lead to a better rooting ratio.

It is sufficient that the auxin solution contain an auxin plant hormone, and any dispersion medium may be used to dissolve the auxin plant hormone, including water, isotonic solutions, buffers, and tissue culture media. Examples of isotonic solutions include liquids prepared by adding an inorganic salt such as KCl, NaCl, CaCl₂, or MgCl₂ to a concentration of 0.01 to 7 M, preferably 0.5 to 2 M. Examples of buffers include phosphate buffer, Tris buffer, and MES buffer. Examples of tissue culture media include the above-mentioned media. To more suitably achieve the advantageous effect, water is preferred among these. In other words, the auxin solution is preferably an aqueous solution in which an auxin plant hormone is dissolved in water.

Although any component may be incorporated in addition to the auxin plant hormone into the auxin solution, glutathione is preferred. This can lead to a better rooting ratio.

The glutathione is a tripeptide composed of glutamic acid, cysteine, and glycine as constituent amino acids and may be reduced glutathione, oxidized glutathione (glutathione disulfide), or a mixture thereof, preferably reduced glutathione.

The glutathione concentration in the auxin solution is preferably at least 10 pmol/L, more preferably at least 30 pmol/L, still more preferably at least 40 pmol/L, particularly preferably at least 60 pmol/L, most preferably at least 80 pmol/L, but is preferably not more than 500 pmol/L, more preferably not more than 400 pmol/L, still more preferably not more than 300 pmol/L, particularly preferably not more than 200 pmol/L, most preferably not more than 150 pmol/L. This can lead to a better rooting ratio.

Plant hormones other than auxin plant hormones may also be used in the auxin solution. Cytokinin plant hormones as described for the cytokinin plant hormones usable in the induction medium may be used in the auxin solution, but preferably no plant hormone other than auxin plant hormones is used in the auxin solution.

The concentration of plant hormones other than auxin plant hormones in the auxin solution is preferably not more than 2.0 mg/L, more preferably not more than 1.0 mg/L, still more preferably not more than 0.1 mg/L, particularly preferably not more than 0.08 mg/L, most preferably 0 mg/L. This can lead to a better rooting ratio.

### (Culture step)

A culture step includes culturing the shoot soaked in the soaking step in a rooting induction medium to cause rooting.

Here, although the rooting induction medium may be a liquid or a solid, solid culture is preferred because rooting is facilitated when the shoot is inserted into and cultured in a medium. Moreover, when the rooting induction medium is a liquid medium, static culture or shake culture may be carried out.

The shoot to be subjected to the culture step may be any shoot that is soaked in the soaking step. In particular, it is preferably a shoot in the base of which a tissue mass is formed by soaking the shoot in the soaking step. Moreover, more preferably a shoot in the base of which a tissue mass is already formed is used in the soaking step.

The rooting induction medium contains a carbon source.

Any plant hormone may be used in the rooting induction medium, including those as described for the plant hormones (auxin plant hormones, cytokinin plant hormones) usable in the induction medium.

Although any carbon source may be used in the rooting induction medium, including those as described for the carbon sources usable in the induction medium, sucrose is preferred among these. This can lead to a better rooting ratio.

Like the induction medium, the rooting induction medium preferably further contains activated carbon and/or silver nitrate. This can lead to a better rooting ratio.

Like the auxin solution, the rooting induction medium preferably further contains glutathione. This can lead to a better rooting ratio.

The glutathione may be reduced glutathione, oxidized glutathione, or a mixture thereof, preferably reduced glutathione.

Examples of the rooting induction medium include those prepared by adding carbon sources to base media such as the basal media usable as the induction medium and modified basal media obtained by altering the composition of the basal media. Preferred among these are those prepared by adding carbon sources to MS medium, B5 medium, WP medium, or MB medium. More preferred are those prepared by adding carbon sources to MS medium, a modified MS medium obtained by altering the composition of the medium, MB medium, or a modified MB medium obtained by altering the composition of the medium. Still more preferred are those prepared by adding carbon sources to MB medium or a modified MB medium obtained by altering the composition of the medium.

When the rooting induction medium is used as a solid medium, the medium may be solidified using a solidifying agent. Any solidifying agent may be used, including Japanese gelatin, gellan gum, agarose, Gelrite, agar, and Phytagel.

The suitable composition and culture conditions of the rooting induction medium vary depending on the plant species and also vary on whether the medium is a liquid medium or a solid medium, but the composition is usually as follows, particularly for rubber trees.

The carbon source concentration in the rooting induction medium is preferably at least 0.1% by mass, more preferably at least 1.0% by mass. The carbon source concentration is preferably not more than 10% by mass, more preferably not more than 6.0% by mass.

The plant hormone concentration in the rooting induction medium is preferably not more than 2.0 mg/L, more preferably not more than 1.0 mg/L, still more preferably not more than 0.1 mg/L, particularly preferably not more than 0.08 mg/L, most preferably 0 mg/L. This can lead to a better rooting ratio.

The activated carbon concentration in the rooting induction medium is preferably at least 0.005% by mass, more preferably at least 0.008% by mass. The activated carbon concentration is preferably not more than 1.0% by mass, more preferably not more than 0.1% by mass.

The silver nitrate concentration in the rooting induction medium is preferably at least 0.1 mg/L, more preferably at least 0.3 mg/L, still more preferably at least 0.5 mg/L. The silver nitrate concentration is preferably not more than 5.0 mg/L, more preferably not more than 3.0 mg/L.

The glutathione concentration in the rooting induction medium is preferably at least 10 pmol/L, more preferably at least 30 pmol/L, still more preferably at least 40 pmol/L, particularly preferably at least 60 pmol/L, most preferably at least 80 pmol/L, but is preferably not more than 500 pmol/L, more preferably not more than 400 pmol/L, still more preferably not more than 300 pmol/L, particularly preferably not more than 200 pmol/L, most preferably not more than 150 pmol/L.

The pH of the rooting induction medium is preferably 4.0 to 10.0, more preferably 5.0 to 6.5, still more preferably 5.5 to 6.0.

The culture step is usually carried out in a controlled environment in which culture conditions such as temperature and light cycle are managed. The culture conditions may be selected as appropriate, but, for example, the culture temperature is preferably 0 to 40°C, more preferably 20 to 40°C, still more preferably 25 to 35°C. Culture may be carried out in the dark or light, but, for example, the light conditions may include a light cycle of 14 to 16 hours light at 5 to 20 µmol/m²/s. The culture duration is not limited, but culture for one to ten weeks is preferred, and culture for four to eight weeks is more preferred.

Herein, the culture duration in the culture step is defined as follows: culture starts (0 hour) when the shoot (shoot segment) soaked in the soaking step is transplanted to the rooting induction medium; a culture period of three weeks and a culture period of nine weeks mean 504 hours and 1512 hours, respectively, from the start of culture; and if the shoot is transplanted (replanted) to a new rooting induction medium, the culture period is not reset but continues to increase.

When the rooting induction medium is a solid medium, the solidifying agent concentration in the rooting induction medium is preferably at least 0.1% by mass, more preferably at least 0.2% by mass. The solidifying agent concentration is preferably not more than 2.0% by mass, more preferably not more than 1.1% by mass, still more preferably not more than 0.75% by mass.

Among the conditions indicated above, it is preferred that the medium has a low plant hormone concentration (substantially no plant hormone) or contains glutathione. It is more preferred that the medium has a low plant hormone concentration (substantially no plant hormone) and further contains glutathione.

As described above, the shoot soaked in the soaking step may be cultured in the rooting induction medium to cause rooting, thereby obtaining a rooted shoot (plantlet). Thus, a complete clonal sapling can be formed.

According to the present invention, the obtained plantlet (woody plant shoot-derived plantlet), which, in the conventional technique, is transplanted directly into soil, is subjected to the following cultivation step.

### (Cultivation step)

A cultivation step includes cultivating the woody plant shoot-derived plantlet in a solid medium containing a medium component.

The plantlet to be subjected to the cultivation step may be any plantlet that is derived from a woody plant shoot. The plantlet to be subjected to the cultivation step encompasses not only a rooted shoot but also a rooted shoot that has shown a tendency of poor growth in usual soil cultivation.

The plantlet to be subjected to the cultivation step preferably has completed leaf development. Herein, the phrase "has completed leaf development" means that the development of leaves emerged from elongated buds has been completed.

The root length of the plantlet to be subjected to the cultivation step is preferably 5.0 cm or less, more preferably 3.0 cm or less, still more preferably 2.0 cm or less, particularly preferably 1.5 cm or less. As the roots may be root primordia, the lower limit of the root length is not limited, but is preferably 0.1 cm or more, more preferably 0.3 cm or more, still more preferably 0.5 cm or more. When the root length is within the range indicated above, the advantageous effect tends to be more suitably achieved. Moreover, as the production method can promote the root growth of, for example, even a very young woody plant shoot-derived plantlet in the initial stage of rooting in vitro, the method can promote the root growth of even a just rooted plantlet which only has roots with a predetermined length or less as indicated above.

Here, the root length of the plantlet to be subjected to the cultivation step refers to the root length of the plantlet at the start of the cultivation step.

The height above ground of the plantlet to be subjected to the cultivation step is preferably 1 m or less, more preferably 50 cm or less, still more preferably 20 cm or less, but is preferably 1.5 cm or more, more preferably 3.0 cm or more. When the height is within the range indicated above, the advantageous effect tends to be more suitably achieved.

Here, the height above ground of the plantlet to be subjected to the cultivation step refers to the height above ground of the plantlet at the start of the cultivation step.

The solid medium may be any medium insoluble in water. Examples include known culture soils, and specific examples include culture soils prepared by appropriately mixing at least one selected from the group consisting of sand, peat moss, activated carbon, dolomite, isolite, bentonite, zeolite, perlite, vermiculite, ceramic, coconut fibers, bark media, rice husks, rock wool, and other various soil improvement materials.

In addition to the above-mentioned culture soils, examples of usable solid media include resin media such as granular, foam, or sponge resins; and media prepared by solidifying liquid media (e.g., below-described basal media) using solidifying agents.

These solid media may be used alone or in combinations of two or more. Moreover, the solid medium used may be uncompressed or may be compressed at an appropriate compression ratio.

Non-limiting examples of the resins include polyethylene resins, polypropylene resins, polystyrene resins, polyurethane resins, phenolic resins, and polyester resins.

Non-limiting examples of the solidifying agents include Japanese gelatin, gellan gum, agarose, Gelrite, gelatin, and silica gel.

The solid medium is preferably a culture soil, more preferably vermiculite, peat moss, or rock wool, still more preferably vermiculite.

The wet density of the solid medium containing a medium component is preferably 0.4 g/cm³ or more, more preferably 0.5 g/cm³ or more, still more preferably 0.6 g/cm³ or more, but is preferably 1.0 g/cm³ or less, more preferably 0.9 g/cm³ or less, still more preferably 0.8 g/cm³ or less. Such a solid medium can moderately stimulate the roots and also contains oxygen, and therefore the advantageous effect tends to be more suitably achieved.

Herein, the wet density of the solid medium containing a medium component refers to the mass per unit volume of the entire solid medium. Specifically, it is measured by a caliper method in accordance with JIS A 1225(2020).

The average particle size of the solid medium containing a medium component is preferably 0.25 mm or more, more preferably 0.5 mm or more, still more preferably 1.0 mm or more, but is preferably 8.0 mm or less, more preferably 6.0 mm or less, still more preferably 4.0 mm or less. Such a solid medium can moderately stimulate the roots, and thus the advantageous effect tends to be more suitably achieved.

Herein, the average particle size of the solid medium containing a medium component refers to the average particle size by mass calculated from the particle size distribution measured in accordance with JIS Z 8815(1994).

Non-limiting examples of the medium component in the solid medium containing a medium component include nitrogen, phosphorus, potassium, calcium, magnesium, other trace elements, vitamins, carbon sources, amino acids, and plant hormones. These may be used alone or in combinations of two or more. Preferably, the medium component(s) includes at least one selected from the group consisting of nitrogen, phosphorus, potassium, calcium, magnesium, other trace elements, vitamins, carbon sources, amino acids, and plant hormones, more preferably at least one selected from the group consisting of carbon sources, phosphorus, potassium, calcium, and amino acids, still more preferably at least one selected from the group consisting of carbon sources, phosphorus, potassium, and calcium, particularly preferably carbon sources. Here, non-limiting examples of the trace elements other than nitrogen, phosphorus, potassium, calcium, and magnesium include copper, manganese, cobalt, zinc, boron, and iron.

Any carbon source may be used, including sugars such as sucrose, glucose, trehalose, fructose, lactose, galactose, xylose, allose, talose, gulose, altrose, mannose, idose, arabinose, apiose, and maltose. These may be used alone or in combinations of two or more. Sucrose is preferred among these.

Non-limiting examples of the vitamins include biotin, thiamine (vitamin B1), pyridoxine (vitamin B4), pyridoxal, pyridoxamine, calcium pantothenate, inositol, nicotinic acid, nicotinic acid amide, and riboflavin (vitamin B2). These may be used alone or in combinations of two or more.

Non-limiting examples of the plant hormones include auxin plant hormones such as 2,4-dichlorophenoxyacetic acid, 1-naphthaleneacetic acid, indole-3-butyric acid, indole-3-acetic acid, indolepropionic acid, chlorophenoxyacetic acid, naphthoxyacetic acid, phenylacetic acid, 2,4,5-trichlorophenoxyacetic acid, para-chlorophenoxyacetic acid, 2-methyl-4-chlorophenoxyacetic acid, 4-fluorophenoxyacetic acid, 2-methoxy-3,6-dichlorobenzoic acid, 2-phenyl acid, picloram, and picolinic acid; and cytokinin plant hormones such as benzyladenine, kinetin, zeatin, benzylaminopurine, isopentenylaminopurine, thidiazuron, isopentenyladenine, zeatin riboside, and dihydrozeatin. These may be used alone or in combinations of two or more.

The carbon source concentration in the solid medium containing a medium component is preferably at least 0.1% by mass, more preferably at least 0.5% by mass, still more preferably at least 1.0% by mass. The carbon source concentration is preferably not more than 10% by mass, more preferably not more than 8.0% by mass, still more preferably not more than 6.0% by mass. When the concentration is within the range indicated above, the advantageous effect tends to be more suitably achieved. Herein, the term "carbon source concentration" refers to the concentration of sugars.

The solid medium containing a medium component is preferably a medium obtained by adding a liquid medium to the above-described solid medium.

Non-limiting examples of the liquid medium include basal media such as White's medium (disclosed on pp. 20-36 of Shokubutsu Saibo Kogaku Nyumon (Introduction to Plant Cell Engineering), Japan Scientific Societies Press), Heller's medium (Heller R, Bot. Biol. Veg. Paris 14, 1-223 (1953)), SH medium (Schenk and Hildebrandt medium), MS medium (Murashige and Skoog medium) (disclosed on pp. 20-36 of Shokubutsu Saibo Kogaku Nyumon (Introduction to Plant Cell Engineering), Japan Scientific Societies Press), LS medium (Linsmaier and Skoog medium) (disclosed on pp. 20-36 of Shokubutsu Saibo Kogaku Nyumon (Introduction to Plant Cell Engineering), Japan Scientific Societies Press), Gamborg medium, B5 medium (disclosed on pp. 20-36 of Shokubutsu Saibo Kogaku Nyumon (Introduction to Plant Cell Engineering), Japan Scientific Societies Press), MB medium (disclosed on pp. 222-245 of Biotechnology in Agriculture and Forestry volume 5 (TreesII)), and WP medium (for woody plants), and modified basal media obtained by altering the composition of the foregoing basal media (the disclosure of each of the foregoing documents is herein incorporated by reference). These may be used alone or in combinations of two or more. In particular, the liquid medium is preferably a basal medium or a modified basal medium, more preferably a modified basal medium, still more preferably a 1/2 basal medium having half the component content of a basal medium, particularly preferably 1/2 MS medium, because these media contain nutrients necessary for growth of plants, and the advantageous effect can be more suitably achieved.

Moreover, the modified basal medium is also preferably a medium obtained by adding a carbon source to a basal medium or a 1/2 basal medium, more preferably a medium obtained by adding a carbon source to a 1/2 basal medium.

The liquid medium is preferably supplemented with a carbon source.

The carbon source concentration in the liquid medium is preferably at least 0.1% by mass, more preferably at least 1.0% by mass, still more preferably at least 2.0% by mass. The carbon source concentration is preferably not more than 10% by mass, more preferably not more than 7.5% by mass. When the carbon source concentration is within the range indicated above, the advantageous effect tends to be more suitably achieved. Herein, the term "carbon source concentration" refers to the concentration of sugars.

Although the amount of the liquid medium added to the solid medium is not limited, the amount per 100 parts by mass of the solid medium is preferably 10 parts by mass or more, more preferably 20 parts by mass or more, still more preferably 30 parts by mass or more, but is preferably 95 parts by mass or less, more preferably 90 parts by mass or less, still more preferably 85 parts by mass or less. When the amount is within the range indicated above, the advantageous effect tends to be more suitably achieved.

In the cultivation step, the rooting and root growth and development are promoted. Thus, to more suitably achieve this advantageous effect and to be well taprooted, the depth of the solid medium containing a medium component is preferably large. Specifically, the cultivation step preferably includes cultivating the plantlet in an incubator filled with the solid medium containing a medium component to a depth of 10 cm or more, more preferably includes cultivating the plantlet in an incubator filled with the solid medium containing a medium component to a depth of 15 cm or more. The upper limit of the depth is not limited.

In the cultivation step, cultivation is preferably carried out in an environment with an illuminance at the leaf level in the light of 5000 lx or higher. The illuminance is preferably 6000 lx or higher, more preferably 7000 lx or higher, but is preferably 15000 lx or lower, more preferably 10000 lx or lower. When the illuminance is within the range indicated above, the advantageous effect tends to be more suitably achieved.

Herein, the illuminance at the leaf level is measured in accordance with JIS C 7612.

The light source used to obtain the above-mentioned illuminance is not limited and may be natural light, artificial light, or a combination thereof. When artificial light is used, examples include light-emitting diodes (LEDs), halogen lamps, incandescent lamps, fluorescent lamps, arc lamps, electrodeless discharge lamps, low-voltage discharge lamps, cold cathode fluorescent tubes, external electrode fluorescent lamps, electroluminescent lights, and HID lamps. Examples of HID lamps include high-pressure mercury lamps, metal halide lamps, and high-pressure sodium lamps. These light sources may be used alone or in combinations of two or more.

Although the photoperiod (in the light) in the cultivation step is not limited, the photoperiod is preferably 12 hours or longer, more preferably 14 hours or longer, but is preferably 22 hours or shorter, more preferably 20 hours or shorter. When the photoperiod is within the range indicated above, the advantageous effect tends to be more suitably achieved.

The cultivation temperature in the cultivation step is preferably 20°C or higher, more preferably 22°C or higher, still more preferably 24°C or higher, particularly preferably 26°C or higher, but is preferably 36°C or lower, more preferably 34°C or lower, still more preferably 32°C or lower, particularly preferably 30°C or lower. When the cultivation temperature is within the range indicated above, the advantageous effect tends to be more suitably achieved.

Although the cultivation period in the cultivation step is not limited, the cultivation period is preferably one week or longer, more preferably two weeks or longer, still more preferably three weeks or longer, and the upper limit is not limited. When the cultivation period is within the range indicated above, the advantageous effect tends to be more suitably achieved.

Other cultivation conditions are not limited and cultivation may be carried out under usual conditions suitable for plant growth. Moreover, systems and the like for cultivation are also not limited, and those usually used in soil cultivation may be used.

As described above, with a cultivation step which includes cultivating a woody plant shoot-derived plantlet in a solid medium containing a medium component, rooting and root development, which tend to be slower than the growth of the above-ground parts, may be promoted to provide a good balance between the growth of the above-ground parts and root growth, and thus it is possible to produce a sapling of a woody plant (a clonal sapling as a complete plant) from a woody plant shoot-derived plantlet with high productivity.

The produced sapling (plant) may be transplanted into soil, if necessary.

Moreover, acclimatization is also completed in the cultivation step. Thus, the cultivation step can efficiently promote both acclimatization and initial growth, and therefore it is possible to produce a plant (a clonal sapling as a complete plant) from a woody plant shoot-derived plantlet with high productivity, without the need to perform a separate acclimatization step.

### <Method for producing natural rubber>

The method for producing a natural rubber of the present invention includes:
a plant production step of producing a sapling (plant) by the method for producing a sapling of a woody plant; and
a natural rubber production step of producing a natural rubber using the sapling (plant) obtained in the plant production step. The method for producing a natural rubber of the present invention includes a plant production step of producing a sapling (plant) by the method for producing a sapling of a woody plant and thus enables the production of a natural rubber with high productivity.

The plant production step includes producing a sapling (plant) by the method for producing a sapling of a woody plant and may be carried out by performing the method for producing a sapling of a woody plant. If necessary, the sapling (plant) obtained in the method for producing a sapling of a woody plant may be further cultivated into a larger plant.

In the natural rubber production step, a natural rubber is produced using the sapling (plant) obtained in the plant production step. Specifically, a natural rubber may be produced by cultivating the sapling (plant) obtained in the plant production step to biosynthesize a natural rubber in the laticifer cells of the plant.

The natural rubber may be collected from the plant by conventionally known methods.

For example, the plant may be physically cut with a tool such as a knife to collect latex, optionally followed by solidifying the latex, for example, by adding an acid, thereby collecting rubber (natural rubber) as solids from the plant. The thus obtained rubber (natural rubber) may optionally be washed, dehydrated, and dried before use.

### <Method for producing rubber product>

The method for producing a rubber product of the present invention includes: a natural rubber production step of producing a natural rubber by the method for producing a natural rubber; a kneading step of kneading the natural rubber obtained in the natural rubber production step with an additive to obtain a kneaded mixture; a raw rubber product formation step of forming a raw rubber product from the kneaded mixture; and a vulcanization step of vulcanizing the raw rubber product. The method for producing a rubber product of the present invention includes producing a natural rubber by the method for producing a natural rubber and thus enables the production of a rubber product with high productivity.

The rubber product may be any rubber product that can be produced from rubber, preferably natural rubber, and examples include pneumatic tires, rubber crawlers, and rubber fenders.

When the rubber product is a pneumatic tire or, in other words, when the method for producing a rubber product of the present invention is the method for producing a pneumatic tire of the present invention, the raw rubber product formation step corresponds to the green tire formation step of forming a green tire from the kneaded mixture, and the vulcanization step corresponds to the vulcanization step of vulcanizing the green tire. Thus, the method for producing a pneumatic tire of the present invention includes: a natural rubber production step of producing a natural rubber by the method for producing a natural rubber; a kneading step of kneading the natural rubber obtained in the natural rubber production step with an additive to obtain a kneaded mixture; a green tire formation step of forming a green tire from the kneaded mixture; and a vulcanization step of vulcanizing the green tire. The method for producing a pneumatic tire of the present invention includes producing a natural rubber by the method for producing a natural rubber and thus enables the production of a pneumatic tire with high productivity.

The step of producing a natural rubber by the method for producing a natural rubber may be carried out by performing the method for producing a natural rubber to produce a natural rubber.

### <Kneading step>

In the kneading step, the natural rubber obtained in the step of producing a natural rubber by the method for producing a natural rubber is kneaded with an additive to obtain a kneaded mixture.

Any additive may be used including those used in the production of rubber products. For example, when the rubber product is a pneumatic tire, examples of the additive include rubber components other than the rubber obtained from latex, reinforcing fillers such as carbon black, silica, calcium carbonate, alumina, clay, and talc, silane coupling agents, zinc oxide, stearic acid, processing aids, various types of antioxidants, softeners such as oils, waxes, vulcanizing agents such as sulfur, and vulcanization accelerators.

The kneading in the kneading step may be carried out using a rubber kneading machine such as an open roll mill, a Banbury mixer, or an internal mixer.

### <Raw rubber product formation step (green tire formation step in the case of tire)>

In the raw rubber product formation step, a raw rubber product (green tire in the case of tire) is formed from the kneaded mixture obtained in the kneading step.

The method for forming a raw rubber product is not limited, and methods used in the formation of raw rubber products may be used appropriately. For example, when the rubber product is a pneumatic tire, the kneaded mixture obtained in the kneading step may be extruded into the shape of a tire component and then formed and assembled with other tire components in a usual manner on a tire building machine to form a green tire (unvulcanized tire).

### <Vulcanization step>

In the vulcanization step, the raw rubber product obtained in the raw rubber product formation step is vulcanized to produce a rubber product.

The method for vulcanizing the raw rubber product is not limited, and methods used in the vulcanization of raw rubber products may be used appropriately. For example, when the rubber product is a pneumatic tire, the green tire (unvulcanized tire) obtained in the raw rubber product formation step is vulcanized by heating and pressing in a vulcanizer to produce a pneumatic tire.

### <Hevea brasiliensis sapling>

The sapling of a woody plant produced by the above-described production method has a good balance between the growth of the above-ground parts and root growth, and its main root and lateral roots also develop well. Thus, the sapling shows a high percentage of survival in soil and high growth in the young sapling stage.

Although *Hevea brasiliensis,* in particular, is a poor-rooting plant among woody plants, the *Hevea brasiliensis* sapling produced by the production method, unlike conventional *Hevea brasiliensis* saplings, shows a high percentage of survival in soil and high growth in the young sapling stage.

Specifically, the *Hevea brasiliensis* sapling of the present invention has no joint in the vicinity of the root base and is taprooted in the vicinity of the root base, and the *Hevea brasiliensis* sapling of the present invention has a ratio of the height above ground to the root length of 1:1 or more. Such a *Hevea brasiliensis* sapling can be produced by the above-described production method. Specifically, as the production method can produce a sapling from a shoot of *Hevea brasiliensis,* the sapling has no joint in the vicinity of the root base. Moreover, as the production method can promote rooting and root development, the sapling is taprooted in the vicinity of the root base. Furthermore, as the production method allows rooting and root development, which tend to be slower than the growth of the above-ground parts, to be promoted to provide a good balance between the growth of the above-ground parts and root growth, the sapling has a ratio of the height above ground to the root length of 1:1 or more. Here, the *Hevea brasiliensis* sapling of the present invention may also be produced by subjecting a sapling produced by the production method to further cultivation such as usual soil cultivation.

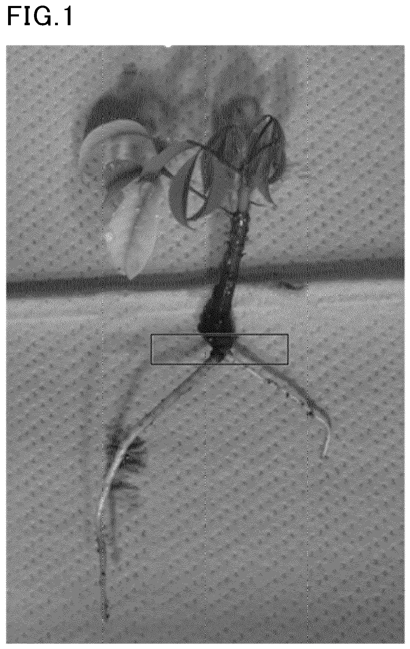

Herein, the term "in the vicinity of the root base" refers to a location in the vicinity of a site where rooting is observed, specifically, a boundary between the stem and root. FIG. 1 is a photograph showing one example in the vicinity of the root base. In FIG. 1, the area enclosed by the rectangle corresponds to a location in the vicinity of the root base.

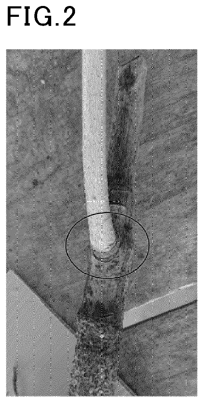

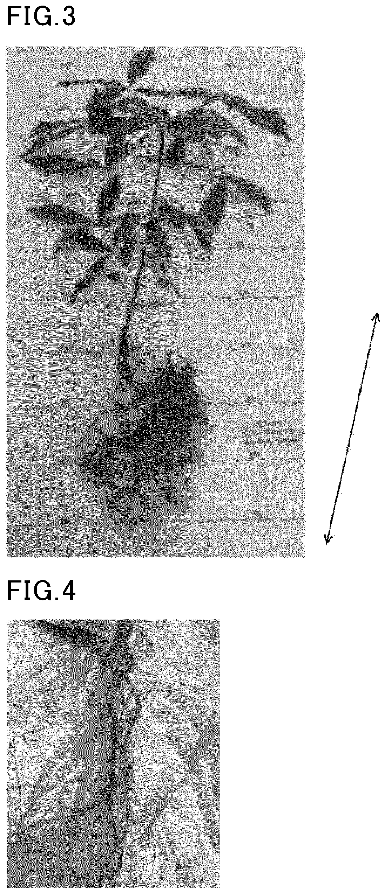

Herein, the term "no joint" means no joining of tissues of two or more plants, e.g., by grafting. FIG. 2 is a photograph showing one example having a joint (in FIG. 2, the area enclosed by the circle corresponds to a joint), and FIG. 3 is a photograph showing one example having no joint. Here, when there is a joint, a scar such as a grafting mark or a bulge may be present in the vicinity of the joint. This bulge is also called an elephant foot or a grafting node and refers to a hump-like or other bulge caused by grafting to a rootstock.

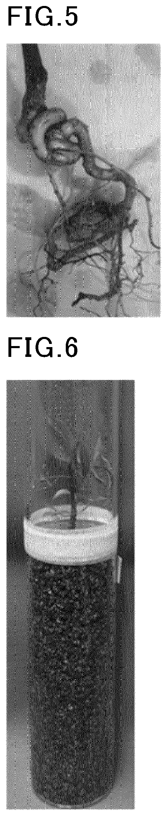

Herein, the expression "taprooted in the vicinity of the root base" means not twisted or spiral in the vicinity of the root base. Conversely, "twisted or spiral in the vicinity of the root base" as used herein means that it is not taprooted in the vicinity of the root base. FIG. 4 is a photograph showing one example which is taprooted in the vicinity of the root base, and FIG. 5 is a photograph showing one example which is not taprooted in the vicinity of the root base. In FIG. 5, the root is spiral.

The expression "taprooted in the vicinity of the root base" specifically means that the angle of the root in the vicinity of the root base is preferably 120° or greater, more preferably 135° or greater, still more preferably 150° or greater, particularly preferably 160° or greater, most preferably 165° or greater, even most preferably 180° (the root in the vicinity of the root base extends in the same direction as a direction that extends along the direction in which the stem in the vicinity of the root base extends).

Herein, the term "angle of the root in the vicinity of the root base" refers to an angle in which an angle of 180° is assigned when the root in the vicinity of the root base extends in the same direction as a direction that extends along the direction in which the stem in the vicinity of the root base extends. For example, the angle is 90° when the root in the vicinity of the root base extends in a direction perpendicular to a direction that extends along the direction in which the stem in the vicinity of the root base extends.

Although the *Hevea brasiliensis* sapling has a ratio of the height above ground to the root length (height above ground:root length) of 1:1 or more, the ratio is preferably 1:1.2 or more, more preferably 1:1.5 or more, but is preferably 1:5 or less, more preferably 1:3 or less, still more preferably 1:2 or less. When the ratio is within the range indicated above, the advantageous effect tends to be more suitably achieved.

Moreover, the root length of the *Hevea brasiliensis* sapling is preferably 3 cm or more, more preferably 10 cm or more, still more preferably 15 cm or more. The upper limit is not limited but is preferably 100 cm or less, more preferably 85 cm or less, still more preferably 70 cm or less. When the root length is within the range indicated above, the advantageous effect tends to be more suitably achieved.

Herein, the term "root length" does not refer to the length determined when the roots are stretched by applying a force but refers to the length from the boundary between the stem and root to the tip of the root when the plant is laid in a natural state on a flat surface such as a plate.

Likewise, herein, the term "height above ground" does not refer to the length determined when the above-ground parts are stretched by applying a force but refers to the length from the boundary between the stem and root to the tip of the above-ground part when the plant is laid in a natural state on a flat surface such as a plate.

Here, the phrase "when the plant is laid in a natural state on a flat surface such as a plate" means that the plant is laid on a flat surface such as a plate while no force (tension or other force) is applied to the plant (for example, in a state as shown in FIG. 3), and the term "length from the boundary between the stem and root to the tip of the root" and the term "length from the boundary between the stem and root to the tip of the above-ground part" refer to the respective lengths in a direction as shown by the arrow in FIG. 3 (in a direction that extends along the direction in which the stem in the vicinity of the root base extends) determined in a state as shown in FIG. 3.

The *Hevea brasiliensis* sapling preferably has a main root.

For *Hevea brasiliensis,* which is a poor-rooting plant, as described earlier, it is very difficult to produce a rooted cutting. Even if produced, the rooted cutting has no main root and shows a very low percentage of survival in soil and thus has poor practical utility as a sapling.

In contrast, the *Hevea brasiliensis* sapling produced by the production method has a good balance between the growth of the above-ground parts and root growth, and further its main root and lateral roots also develop well. In other words, owing to the main root, the *Hevea brasiliensis* sapling shows a high percentage of survival in soil and high growth in the young sapling stage.

Herein, the term "main root" refers to a thick root which is the main part of the root system.

The above-ground parts and roots of the *Hevea brasiliensis* sapling are preferably genetically identical to each other.

In general, *Hevea brasiliensis* saplings are prepared by grafting. Since grafted saplings are prepared by grafting to a plant as a rootstock, the above-ground parts and roots are not genetically identical to each other. In contrast, the above-ground parts and roots of the above-described *Hevea brasiliensis* sapling can be identical to each other, so that the *Hevea brasiliensis* sapling is not affected by a rootstock and also has no joint to provide a sapling with good conductivity.

Herein, the expression "the above-ground parts and roots are genetically identical to each other" means that no grafting is performed.

The *Hevea brasiliensis* sapling is preferably a clonal plant. Such a sapling is not affected by a rootstock and also has no joint to provide a sapling with good conductivity.

The *Hevea brasiliensis* sapling preferably carries at least 99% of the genes of the parent strain, more preferably at least 99.5% of the genes of the parent strain. Such a sapling is not affected by a rootstock and also has no joint to provide a sapling with good conductivity.

Herein, the percentage of the genes of the parent strain carried is determined from the sequence identity of genes.

### EXAMPLES

The present invention is specifically described with reference to examples, but the present invention is not limited thereto.

The chemicals used in the examples are collectively described below.
BA: benzyladenine
KI: kinetin
Silver nitrate: silver nitrate available from Merck
Gelling agent (solidifying agent): Phytagel available from Sigma-Aldrich

### <Induction step>

A tissue including an axillary bud was taken from a sapling of *Hevea brasiliensis.*

Next, the tissue including an axillary bud taken from the sapling was cleaned with running water and then with 70% by mass ethanol, subsequently sterilized with an aqueous sodium hypochlorite solution diluted at approximately 5 to 10% by volume, and washed with sterile water.

Next, the sterilized tissue was inserted into and cultured in an induction medium (solid medium) (induction step). The induction medium was prepared by adding 5.0 mg/L of benzyladenine, 1.0 mg/L of silver nitrate, 6.0% by mass of sucrose, and 0.05% by mass of activated carbon to MB medium, adjusting the pH of the medium to 5.7, and then adding the gelling agent to a concentration of 0.275% by mass, followed by sterilization in an autoclave (121°C, 20 minutes) and cooling in a clean bench.

The *Hevea brasiliensis* tissue was inserted into the induction medium (solid medium) and cultured under a light cycle of 16 hours light at 12.5 µmol/m²/s and at a culture temperature of 28°C to induce shoots. Here, subculture was performed by transplantation to an induction medium having the same composition every four weeks.

The shoots induced in the induction step were used in the following.

A cut end of each shoot was soaked in an auxin solution (5.0 mg/L 1-naphthaleneacetic acid, 5.0 mg/L indole-3-butyric acid, and 100 µmol/L reduced glutathione) for 72 hours (soaking step under a photoperiod of 16 hours light at 12.5 µmol/m²/s and at a temperature of 28°C). Next, the cut end surface of each shoot soaked in the soaking step was inserted into and cultured in a hormone-free solid medium (plant hormone concentration: 0 mg/L, pH 5.7) containing in MB basal medium 3.0% by mass of sucrose, 0.01% by mass of activated carbon, 1.0 mg/L of silver nitrate, 100 µmol/L of reduced glutathione, and 0.275% by mass of the solidifying agent (culture step). The culture was carried out under a photoperiod of 16 hours light at 12.5 µmol/m²/s and at a temperature of 25 to 28°C for eight weeks. Thus, the culture in the hormone-free medium was carried out for the predetermined period to obtain rooted plantlets (shoot-derived plantlets).

Here, the auxin solution was prepared by dissolving the above-mentioned components in distilled water.

Moreover, the medium was prepared by adding all the above-mentioned components except the solidifying agent to the basal medium, adjusting the pH of the medium to 5.7, and then adding the solidifying agent to a concentration of 0.275% by mass, followed by sterilization in an autoclave (121°C, 20 minutes) and cooling in a clean bench.

### (Example 1)

A transparent glass culture test tube was filled with vermiculite and then sterilized in an autoclave at 121°C for 20 minutes. After the sterilization, a liquid medium prepared by adding sucrose to 1/2 MS medium having half the component content of MS medium to a concentration of 3% by mass was added in an amount of 80 parts by mass per 100 parts by mass of the vermiculite. After the addition, the plantlet (height above ground: 4.0 cm, root length: 1.0 cm) was inserted into the surface of the solid medium containing a medium component (vermiculite supplemented with the liquid medium) to plant the plantlet (the wet density of the solid medium containing a medium component: 0.74 g/cm³, the carbon source concentration in the solid medium containing a medium component: 1.3% by mass, the depth of the solid medium containing a medium component: 15 cm). Then, cultivation was carried out under a cycle of 16 hours light and 8 hours dark at a cultivation temperature of 28°C. FIG. 6 is a photograph showing one example of cultivation of a plantlet in a solid medium containing a medium component (vermiculite supplemented with a liquid medium).

Here, the illuminance at the leaf level in the light was maintained at 5,000 to 10,000 lx, and the illuminance at the leaf level in the dark was maintained at 1 lx or lower.

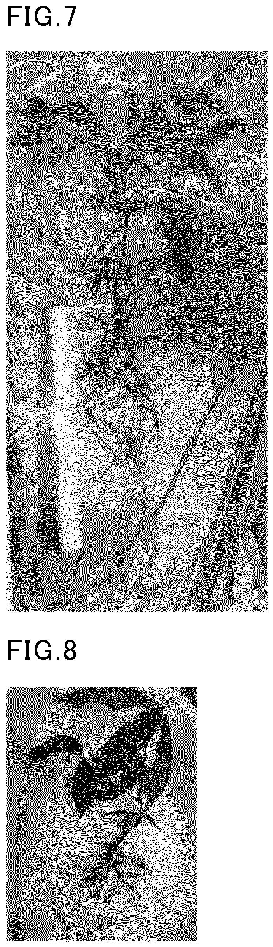

As a result of the cultivation, the root had elongated to a length of approximately 12 cm after a lapse of three weeks and further to a length of approximately 15 cm after a lapse of one month, thereby obtaining a *Hevea brasiliensis* sapling. Then, the sapling was transplanted to a pot containing vermiculite (without any medium component) and cultivated for three months. The *Hevea brasiliensis* sapling produced in Example 1 had no joint in the vicinity of the root base and was taprooted in the vicinity of the root base, the sapling had a main root, and the ratio of the height above ground to the root length was 1:1 or more (root length: 40 cm, height above ground:root length = 1:1.8, angle of root in the vicinity of root base: 165°) (see FIG. 7).

### (Comparative Example 1)

Since the plantlet (root length: 1.0 cm) used in Example 1 died when planted in vermiculite (without any medium component), the plantlet used in Example 1 was replaced with a plantlet (root length: 5.0 cm) prepared by culturing in the hormone-free medium one month longer than for the plantlet used in Example 1. This plantlet (root length: 5.0 cm) was transplanted to a pot containing vermiculite (without any medium component, wet density of solid medium: 1 g/cm³, irrigation with water only) and cultivated for three months under the same conditions as in Example 1 to obtain a *Hevea brasiliensis* sapling (root length: 8 cm, height above ground:root length = 1:0.8, angle of root in the vicinity of root base: 90°) (see FIG. 8). The sapling of Comparative Example 1 was twisted in the vicinity of the root base and was not taprooted in the vicinity of the root base.

### (Comparative Example 2)

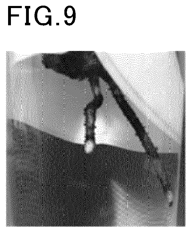

The plantlet (root length: 1.0 cm) was inserted into a liquid medium (without vermiculite) prepared by adding sucrose to 1/2 MS medium having half the component content of MS medium to a concentration of 3% by mass, and then cultivated under the same conditions as in Example 1. As a result, the root had elongated to a length of approximately 5 cm after about 15 days of cultivation but thereafter the root elongation stopped. The root length after three weeks of cultivation was still approximately 5 cm (angle of root in the vicinity of root base: 150°) (see FIG. 9).

Table 1 summarizes the periods from rooting until the root length reached approximately 15 cm and the states of the roots after a lapse of four months from rooting of Example and Comparative Examples 1 and 2. In Table 1, the term "rooting" means the time when the root length reached 1.0 cm.

**[Table 1]**

| | Example | Comparative Example 1 | Comparative Example 2 |
|---|---|---|---|
| Period from rooting until root length reached approximately 15 cm | one month | four months | root growth stopped after reaching 5 cm |
| State of root after lapse of four months from rooting | Root length: 40 cm | Root length: 8 cm | - |
| | lateral roots: many | lateral roots: few | |

As shown, when a woody plant shoot-derived plantlet is cultivated in a solid medium containing a medium component, the rooting and root development, which tend to be slower than the growth of the above-ground parts, may be promoted to provide a good balance between the growth of the above-ground parts and root growth, and thus it is possible to produce a sapling of a woody plant from a woody plant shoot-derived plantlet with high productivity.

Exemplary embodiments of the present invention include:
Embodiment 1. A method for producing a sapling of a woody plant, including
   a cultivation step of cultivating a woody plant shoot-derived plantlet in a solid medium containing at least one medium component.
Embodiment 2. The method for producing a sapling of a woody plant according to Embodiment 1,
   wherein the medium component includes at least one selected from the group consisting of nitrogen, phosphorus, potassium, calcium, magnesium, other trace elements, vitamins, carbon sources, amino acids, and plant hormones.
Embodiment 3. The method for producing a sapling of a woody plant according to Embodiment 1 or 2,
   wherein the solid medium has a wet density of 0.4 to 1.0 g/cm³.
Embodiment 4. The method for producing a sapling of a woody plant according to any combination with any one of Embodiments 1 to 3,
   wherein the solid medium has an average particle size of 0.25 to 8.0 mm.
Embodiment 5. The method for producing a sapling of a woody plant according to any combination with any one of Embodiments 1 to 4,
   wherein the solid medium is a culture soil.
Embodiment 6. The method for producing a sapling of a woody plant according to any combination with any one of Embodiments 1 to 5,
   wherein the cultivation step includes cultivating the plantlet in an incubator filled with the solid medium to a depth of 15 cm or more.
Embodiment 7. The method for producing a sapling of a woody plant according to any combination with any one of Embodiments 1 to 6,
   wherein the woody plant is a plant belonging to the genus *Hevea.*
Embodiment 8. The method for producing a sapling of a woody plant according to any combination with any one of Embodiments 1 to 6,
   wherein the woody plant is *Hevea brasiliensis.*
Embodiment 9. A method for producing a natural rubber, including:
   a plant production step of producing a sapling by the method for producing a sapling according to any combination with any one of Embodiments 1 to 8; and
   a natural rubber production step of producing a natural rubber using the sapling obtained in the plant production step.
Embodiment 10. A method for producing a pneumatic tire, including:
   a natural rubber production step of producing a natural rubber by the method for producing a natural rubber according to Embodiment 9;
   a kneading step of kneading the natural rubber obtained in the production step with an additive to obtain a kneaded mixture;
   a green tire formation step of forming a green tire from the kneaded mixture; and
   a vulcanization step of vulcanizing the green tire.
Embodiment 11. A method for producing a rubber product, including:
   a natural rubber production step of producing a natural rubber by the method for producing a natural rubber according to Embodiment 9;
   a kneading step of kneading the natural rubber obtained in the natural rubber production step with an additive to obtain a kneaded mixture;
   a raw rubber product formation step of forming a raw rubber product from the kneaded mixture; and
   a vulcanization step of vulcanizing the raw rubber product.
Embodiment 12. A *Hevea brasiliensis* sapling,
   having no joint in a vicinity of a root base and being taprooted in the vicinity of the root base, and
   having a ratio of a height above ground to a root length of 1:1 or more.
Embodiment 13. The *Hevea brasiliensis* sapling according to Embodiment 12, which has a main root.
Embodiment 14. The *Hevea brasiliensis* sapling according to Embodiment 12 or 13,
   wherein an above-ground part and a root are genetically identical to each other.
Embodiment 15. The *Hevea brasiliensis* sapling according to any combination with any one of Embodiments 12 to 14, which is a clonal plant.
Embodiment 16. The *Hevea brasiliensis* sapling according to any combination with any one of Embodiments 12 to 15, which carries at least 99% of genes of a parent strain.

## Claims

1. A method for producing a sapling of a woody plant, comprising
a cultivation step of cultivating a woody plant shoot-derived plantlet in a solid medium containing at least one medium component.

2. The method for producing a sapling of a woody plant according to claim 1,
wherein the medium component comprises at least one selected from the group consisting of nitrogen, phosphorus, potassium, calcium, magnesium, other trace elements, vitamins, carbon sources, amino acids, and plant hormones.

3. The method for producing a sapling of a woody plant according to claim 1 or 2,
wherein the solid medium has a wet density of 0.4 to 1.0 g/cm³.

4. The method for producing a sapling of a woody plant according to any one of claims 1 to 3,
wherein the solid medium has an average particle size of 0.25 to 8.0 mm.

5. The method for producing a sapling of a woody plant according to any one of claims 1 to 4,
wherein the solid medium is a culture soil.

6. The method for producing a sapling of a woody plant according to any one of claims 1 to 5,
wherein the cultivation step comprises cultivating the plantlet in an incubator filled with the solid medium to a depth of 15 cm or more.

7. The method for producing a sapling of a woody plant according to any one of claims 1 to 6,
wherein the woody plant is a plant belonging to the genus *Hevea.*

8. The method for producing a sapling of a woody plant according to any one of claims 1 to 6,
wherein the woody plant is *Hevea brasiliensis.*

9. A method for producing a natural rubber, comprising:
a plant production step of producing a sapling by the method for producing a sapling according to any one of claims 1 to 8; and
a natural rubber production step of producing a natural rubber using the sapling obtained in the plant production step.

10. A method for producing a pneumatic tire, comprising:
a natural rubber production step of producing a natural rubber by the method for producing a natural rubber according to claim 9;
a kneading step of kneading the natural rubber obtained in the natural rubber production step with an additive to obtain a kneaded mixture;
a green tire formation step of forming a green tire from the kneaded mixture; and
a vulcanization step of vulcanizing the green tire.

11. A method for producing a rubber product, comprising:
a natural rubber production step of producing a natural rubber by the method for producing a natural rubber according to claim 9;
a kneading step of kneading the natural rubber obtained in the natural rubber production step with an additive to obtain a kneaded mixture;
a raw rubber product formation step of forming a raw rubber product from the kneaded mixture; and
a vulcanization step of vulcanizing the raw rubber product.

12. A *Hevea brasiliensis* sapling,
having no joint in a vicinity of a root base and being taprooted in the vicinity of the root base, and
having a ratio of a height above ground to a root length of 1:1 or more.

13. The *Hevea brasiliensis* sapling according to claim 12, which has a main root.

14. The *Hevea brasiliensis* sapling according to claim 12 or 13,
wherein an above-ground part and a root are genetically identical to each other.

15. The *Hevea brasiliensis* sapling according to any one of claims 12 to 14, which is a clonal plant.

16. The *Hevea brasiliensis* sapling according to any one of claims 12 to 15, which carries at least 99% of genes of a parent strain.
